# EUROPEAN PATENT APPLICATION

(11) **EP 1 938 809 A1**
(43) Date of publication of application: **02.07.2008**
(21) Application number: 06797990.6
(22) Date of filing: 14.09.2006
(51) Int. Cl.: A61K 9/70, A61K 31/125, A61K 31/192, A61K 31/405, A61K 31/618, A61K 47/36, A61K 47/38, A61P 17/00, A61P 29/00

(54) **ADHESIVE SKIN PATCH**

(30) Priority: 20.09.2005 JP 2005272590
(71) Applicant: Hisamitsu Pharmaceutical Co., Inc., Tosu-shi, Saga 841-0017 (JP)
(72) Inventor: TSURU, Seiichiro, Saga 841-0017 (JP); HINOTANI, Tomoyuki, deceased (JP)
(74) Representative: Grünecker, Kinkeldey, Stockmair & Schwanhäusser Anwaltssozietät
(86) International application number: PCT/JP2006/318278
(87) International publication number: WO 2007/034736

(57) **Abstract**

A plaster including a pressure-sensitive adhesive layer containing a readily dissolvable agar and at least one additive selected from the group consisting of polyacrylamides, polyvinyl alcohols, polyvinyl pyrrolidones, sodium alginate, ammonium alginate, carboxymethylcelluloses, sodium carboxymethylcelluloses, methylcelluloses, gum arabics, tragacanth gums, and xanthene rubbers, is provided.

## Description

### Technical Field

The present invention relates to a plaster.

### Background Art

In plasters having a pressure-sensitive adhesive layer containing much moisture, such as poultices and sheet-like packing agents, a pressure-sensitive adhesive base containing a water-soluble polymer such as gelatins, polyacrylic acids or polyacrylates is used for forming the pressure-sensitive adhesive layer. Especially a pressure-sensitive adhesive base containing a gelatin is suitable because it has a high moisture content and an excellent form preservability and cohesiveness. However, since a pressure-sensitive adhesive base containing a gelatin has a pH range where the form preserving capability and the cohesive force of the gelatin are exhibited, there is a problem that the selection range of substances to be formulated has a limit. Further, since a pressure-sensitive adhesive layer formed of a pressure-sensitive adhesive base containing a gelatin has a low melting point of the gelatin, there is also a problem of liquid sagging when the pressure-sensitive adhesive layer is exposed to a high temperature.

Then, a pressure-sensitive adhesive base containing an agar in addition to a gelatin is proposed for the purpose of improving the moisture preservability and the form preservability of the pressure-sensitive adhesive base containing a gelatin (see Patent Document 1). Generally, agars have advantages of having an excellent form preservability because they have a high solidifying point of about 35 to 45°C and do not dissolve nearly at room temperature and also their form moisture preservability not inferior to gelatins.
Patent Document 1: Japanese Patent Laid-Open No. 57-42617
Patent Document 2: Japanese Patent Laid-Open No. 2005-176742

### Disclosure of the Invention

### Problem to be Solved by the Invention

However, there is a problem of hindering the efficient manufacturing when a plaster having a pressure-sensitive adhesive layer formed of the pressure-sensitive adhesive base of Patent Document 1 is manufactured. First, since common agars have a high melting point of about 80°C, the agars must be heated at not less than 90°C to be dissolved. Hence, the agars have a disadvantage that containing ingredients weak in heat and volatile ingredients is not suitable.

Commonly, a plaster having a pressure-sensitive adhesive layer containing an agar can be manufactured by heating and dissolving an agar powder in water, further adding formulation substances thereto, stirring the mixture under heating to obtain a pressure-sensitive adhesive, and then applying the agent on a release sheet or a support. Since agars have a higher solidifying point (35 to 45°C) than that of gelatins, although gelatins do not gelatinize without being cooled below room temperature, the agars begin to gelatinize when the temperature of their solution becomes about 45°C. If an agar-containing liquid has gelatinized on the way of a manufacturing process, it is unpreferable because the liquid cannot be used in a post process. For preventing this, manufacture by keeping the whole manufacturing facility at not less than 45°C is conceivable, but it is problematically unpreferable and inefficient in view of saving costs, time, labor hours and energy.

Patent Document 2 recited above shows an example in which the gelation temperature of an agar-containing liquid is decreased. The patent document shows that a solution obtained by heating and dissolving a modified agar obtained by mixing an agar with a cyclodextrin has a more decreased gelation temperature (solidifying point) than that of a common agar-containing liquid.

In manufacture of plasters containing not only medicinal ingredients and cosmetic ingredients but many other ingredients, a simple and concise method has been demanded in which an agar is dissolved at a low temperature and the gelation temperature is lowered. The conventional technology described above does not sufficiently meet such a necessity and does not provide means to efficiently manufacture an agar-containing plaster.

Therefore, it is an object of the present invention to provide a plaster having a pressure-sensitive adhesive layer which dissolves at a lower temperature and does not gelatinize till a lower gelation temperature on manufacturing by a simple and concise means.

### Means for Solving the Problem

To achieve the above object, the present invention provides a plaster including a support and a pressure-sensitive adhesive layer disposed on at least one surface of the support, wherein the pressure-sensitive adhesive layer contains a readily dissolvable agar and at least one additive selected from the group consisting of polyacrylamides, polyvinyl alcohols, polyvinyl pyrrolidones, sodium alginate, ammonium alginate, carboxymethylcelluloses, sodium carboxymethylcelluloses, methylcelluloses, gum arabics, tragacanth gums, and xanthene rubbers, and substantially contains no gelatin.

A "readily dissolvable agar" used in the present invention means an agar nearly 100% of which dissolves at a temperature of 70°C. As long as an agar has such a property, the composition, molecular weight, form, jelly strength, sol viscosity, solidifying point, pH, water absorbing property and syneresis of the agar are not especially limited. Further, in the present invention, "substantially contain no gelatin" means that the plaster contains no gelatin or that even if the plaster contains gelatin, its amount is an amount of a level not influencing the jelly strength of a pressure-sensitive adhesive layer formed of a pressure-sensitive adhesive base. Here, whether or not the amount influences the jelly strength can be judged by the following method. That is, with respect to the jelly strength of a pressure-sensitive adhesive layer formed of a pressure-sensitive adhesive base, if there is no rise or change in the jelly strength of the pressure-sensitive adhesive layer formed of the pressure-sensitive adhesive base containing an amount of a gelatin, the amount is judged not to influence the jelly strength of a pressure-sensitive adhesive layer, and the gelatin of the amount can be regarded as "substantially contain no gelatin". In comparison of the jelly strengths, their differences should be confirmed in consideration of measuring errors.

In the present invention, use of a readily dissolvable agar in a pressure-sensitive adhesive layer allows dissolving the agar at a temperature of about 70°C for about 10 to 15 min on manufacture. As a result, volatile ingredients and thermally-weak ingredients can be contained in a plaster. At least one additive selected from the group consisting of polyacrylamides, polyvinyl alcohols, polyvinyl pyrrolidones, sodium alginate, ammonium alginate, carboxymethylcelluloses, sodium carboxymethylcelluloses, methyl celluloses, gum arabics, tragacanth gums, and xanthene rubbers, decreases the solidifying point of the readily dissolvable agar by an interaction with the readily dissolvable agar. Thereby, a plaster can be efficiently manufactured. The reason of the decrease in the solidifying point of a readily dissolvable agar is not necessarily clear, but the additive described above is believed to function as a gelation preventing agent.

In the present invention, the solidifying point refers to a temperature at which a sol solution gelatinizes by being cooled. The melting point refers to a temperature at which a solidified gel redissolves by being heated.

The additive is preferably composed of at least one selected from the group consisting of polyacrylamides, polyvinyl alcohols and polyvinyl pyrrolidones.

The pressure-sensitive adhesive layer preferably contains 0.1 to 20% by mass of an additive based on the total mass of the pressure-sensitive adhesive layer.

Since the pressure-sensitive adhesive layer contains not less than 0.1 % by mass of an additive, an effect on lowering the solidifying point of a pressure-sensitive adhesive base (referring to a composition forming the pressure-sensitive adhesive layer) can be securely exhibited. By contrast, since the pressure-sensitive adhesive layer contains not more than 20% by mass of the additive, the additive can be prevented from not dissolving and the pressure-sensitive adhesive base can be prevented from not solidifying.

The pressure-sensitive adhesive layer preferably contains 0.1 to 5.0% by mass of a readily dissolvable agar based on the total mass of the pressure-sensitive adhesive layer. Since a pressure-sensitive adhesive layer contains not less than 0.1 % by mass of a readily dissolvable agar, the occurrence of bleeding can be prevented. By contrast, since the pressure-sensitive adhesive layer contains not more than 5.0% by mass of the readily dissolvable agar, the form preservability can be sufficiently kept.

The formulation ratio of a readily dissolvable agar (mass: A) and an additive (mass: B) is preferably A:B=1:0.5 to 1:10. That is, B/A is preferably 0.5 to 10. With B/A of not less than 0.5, an effect on decreasing the solidifying point of a pressure-sensitive adhesive base can be securely exhibited. By contrast, with B/A of not more than 10, the additive can be prevented from not dissolving and the pressure-sensitive adhesive base can be prevented from not solidifying.

The readily dissolvable agar preferably has a jelly strength of 450 to 1,000 g/cm².

In the present invention, the "jelly strength" (unit: g/cm²) refers to a maximum weight (gram number) which a gel can withstand for 20 sec per square centimeter of the surface of the gel obtained by preparing a 1.5-mass% agar aqueous solution and being allowed to stand at 20°C for 15 hours for solidification.

With the jelly strength of the readily dissolvable agar of not less than 450 g/cm², the bleeding of a pressure-sensitive adhesive layer and the residual of the pressure-sensitive adhesive layer can be prevented. Here, the "bleeding of a pressure-sensitive adhesive layer" refers to a phenomenon that the form preservability of a formed pressure-sensitive adhesive layer decreases and the pressure-sensitive adhesive layer migrates to a support and bleeds from the support; the "residual of a pressure-sensitive adhesive layer" refers to a phenomenon that when a plaster pasted on an adherend is peeled off, a part of the pressure-sensitive adhesive layer remains on the adherend. By contrast, with the jelly strength of not more than 1,000 g/cm², the cohesive force of a formed pressure-sensitive adhesive layer increases and the adhesive force of the pressure-sensitive adhesive layer can be prevented from being insufficient.

### Effects of the Invention

According to the present invention, a plaster having a pressure-sensitive adhesive layer which dissolves at a lower temperature and does not gelatinize till a lower gelation temperature on manufacture can be provided by a simpler and more concise means.

### Brief Description of the Drawings

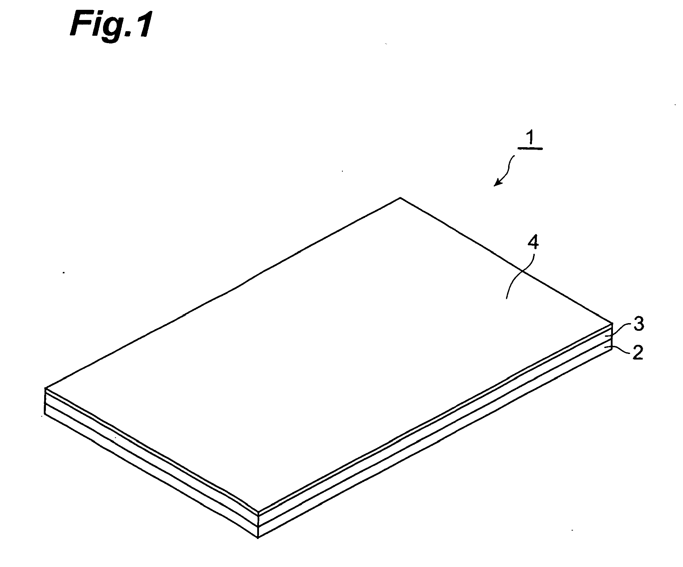

Figure 1 is a perspective view showing a suitable embodiment of the plaster of the present invention.

### Explanation of Reference Numerals

1 ... plaster, 2 ... support, 3 ... pressure-sensitive adhesive layer, 4 ... release sheet.

### Best Modes for Carrying Out the Invention

Hereinafter, an embodiment of the present invention will be described referring to an accompanying drawing.
Figure 1 is a perspective view showing a suitable embodiment of the plaster of the present invention. In Figure 1, a plaster 1 includes a support 2, a pressure-sensitive adhesive layer 3 laminated on the support 2, and a release sheet 4 pasted on the pressure-sensitive adhesive layer 3.

The pressure-sensitive adhesive layer 3 which the plaster 1 of the embodiment has is formed of a pressure-sensitive adhesive base containing a readily dissolvable agar and additives.

Use of a readily dissolvable agar for a pressure-sensitive adhesive base causes the pressure-sensitive adhesive base to dissolve at about 70°C for a heating time of about 10 to 15 min on manufacture. Therefore, volatile ingredients and thermally weak ingredients can be contained in the pressure-sensitive adhesive base. Further, the pressure-sensitive adhesive base has fewer disadvantages (restriction of pH range, liquid sagging), and exhibits a more excellent adhesive force and a reduced residual than plasters in which a gelatin is formulated. Additionally, since the gelation temperature of the pressure-sensitive adhesive base is reduced, the gelation does not occur at undesired times even if a manufacturing facility is not heated. Therefore, a plaster can be easily manufactured and the cost reduction and the promotion of the efficiency are possible.

In the embodiment, the plaster is preferably an aqueous plaster. An "aqueous plaster" means one which contains moisture in its pressure-sensitive adhesive layer. Specifically, poultices, cooling sheets and packing agents are included.

A readily dissolvable agar in the embodiment is preferably an agar from which agaropectin, which hardly dissolves even at high temperatures, is almost removed. Agaropectin contains many ionic functional groups and almost no gelation capability. For dissolving a common agar containing agaropectin, heating the agar in the boiling state for at least 10 to 15 min is needed, which is unsuitable for the agar to contain volatile ingredients and thermally decomposing ingredients. In the embodiment of the present invention, a readily dissolvable agar which can be dissolved at a low temperature is preferably used.

The jelly strength of a readily dissolvable agar used in the embodiment is preferably 450 to 1,000 g/cm², more preferably 500 to 900 g/cm², most preferably 600 to 800 g/cm². With the jelly strength of a readily dissolvable agar of not less than 450 g/cm², the bleeding of a pressure-sensitive adhesive layer and the residual of the pressure-sensitive adhesive layer can be prevented. By contrast, with the jelly strength of not more than 1,000 g/cm², the cohesive force of the pressure-sensitive adhesive layer 3 increases and the adhesive force of the pressure-sensitive adhesive layer 3 can be prevented from being insufficient.

As a readily dissolvable agar used in the embodiment, commercially available ones may be used. Commercially available products include, for example, UP-37K (made by Ina Food Industry Co., Ltd., its jelly strength: 700 g/cm²), UP-26K (made by Ina Food Industry Co., Ltd., its jelly strength: 650 g/cm²), UP-16K (made by Ina Food Industry Co., Ltd., its jelly strength: 600 g/cm²), UZ-5K (made by Ina Food Industry Co., Ltd., its jelly strength: 420 g/cm²) and UM-11K (made by Ina Food Industry Co., Ltd., its jelly strength: 1,000 g/cm²).

The formulation amount of the readily dissolvable agar of the embodiment is preferably 0.1 to 5.0% by mass, more preferably 0.2 to 2.5% by mass. With the contained readily dissolvable agar of not less than 0.1 % by mass, the occurrence of bleeding can be prevented. By contrast, with the readily dissolvable agar of not more than 5.0% by mass, the form preservability of the pressure-sensitive adhesive layer 3 can be fully kept. When a pressure-sensitive adhesive layer is formed from a pressure-sensitive adhesive base, and in the case where contained ingredients do not volatilize, the formulation amount of a pressure-sensitive adhesive layer in terms of total mass is the same as that of a pressure-sensitive adhesive base.

In the embodiment, additives are added when a pressure-sensitive adhesive base containing a readily dissolvable agar is prepared, and the solidifying point of the readily dissolvable agar decreases due to the interaction with the readily dissolvable agar. These additives are preferably water-soluble polymers having a high polarity. They include, for example, polyacrylamides, polyvinyl alcohols, polyvinylpyrrolidones, sodium alginates of polysaccharides, ammonium alginate, carboxymethylcelluloses, sodium carboxymethylcelluloses, methylcelluloses, gum arabics, tragacanth gums and xanthene rubbers. An additional effect of them involves a function as a base agent for the pressure-sensitive adhesive layer 3. Among them, polyvinyl alcohols, polyacrylamides and polyvinylpyrrolidones are preferable. The formulation amount of the additive is preferably 0.1 to 20% by mass, more preferably 0.5 to 10% by mass. With the contained additive of not less than 0.1 % by mass, an effect of more lowering the solidifying point of a pressure-sensitive adhesive base can securely be exhibited. By contrast, with the additive of not more than 20% by mass, the additive can be prevented from not dissolving and the pressure-sensitive adhesive base can be prevented from not solidifying.

The ratio (A:B) of the formulation amounts of a readily dissolvable agar (mass: A) and an additive (mass: B) in the pressure-sensitive adhesive layer 3 is preferably 1:0.1 to 1:50, more preferably 1:0.5 to 1:20, still more preferably 1:0.5 to 1:10. That is, B/A is preferably 0.5 to 10, more preferably 0.5 to 20, still more preferably 0.5 to 10. With B/A of not less than 0.5, an effect of more lowering the solidifying point of a pressure-sensitive adhesive base can securely be exhibited. By contrast, with B/A of not more than 10, the additive can be prevented from not dissolving and the pressure-sensitive adhesive base can be prevented from not solidifying.

Further, from the view point of improving the adhesive force of the pressure-sensitive adhesive layer 3, B/A is preferably 1 to 8.3, more preferably 1 to 1.7.

In the embodiment, the action of reducing the gelation temperature by an additive is not clear, but the present inventors presume as follows. Agarose and agaropectin, which are polysaccharides of constituting ingredients of agars, exist in a random coil state in a solution state, but change into a double helix structure by cooling, and further structure a three-dimensional network and gelatinize. Additives in the embodiment are water-soluble polymers having a high polarity. The additive is believed to reduce the gelation temperature by disturbing the formation of the double helix structure in some mechanism by polysaccharides in an agar and obstructing the structuring of the three-dimensional network, for example, when the sol solution of the agar is cooled.

That the pressure-sensitive adhesive base disclosed in the embodiment contains a readily dissolvable agar and an additive in specific amounts and proportions reduces the gelation temperature of an agar. Hence, even if a manufacturing apparatus and the like are not kept warm at not less than 45°C when the plaster 1 is manufactured, the agar does not gelatinize on the way before a process where the pressure-sensitive adhesive base is applied on the support 2, and the plaster 1 can be efficiently manufactured. Further, since the readily dissolvable agar has a low dissolving temperature, volatile ingredients and thermally decomposing ingredients can be formulated in the pressure-sensitive adhesive base with no loss, as would be difficult with common agars.

Other polymeric compounds may be contained in the pressure-sensitive adhesive layer 3 of the plaster 1 of the embodiment. Such polymeric compounds include, for example, gelatins, collagens, pectins, starches, chitins, albumins, caseins, polyacrylic acids and/or polyacrylate derivatives, carboxyvinyl polymers, vinylpyrrolidone-acrylic acid copolymers, vinylpyrrolidone-styrene copolymers, vinylpyrrolidone-vinyl acetate copolymers, vinyl acetate-(meth)acrylic acid copolymers, polyvinyl acetate-crotonic acid copolymers, N-vinylacetamide copolymers, polyvinylsulfonic acids, polyitaconic acids, polyhydroxyethyl acrylates, styrene-maleic anhydride copolymers and acrylamide-acrylic acid copolymers. Among them, polyacrylic acid derivatives and/or polyacrylate derivatives are preferable and particularly polyacrylic acids and/or polyacrylates are preferable.

Medicinal ingredients, cosmetic ingredients and generally formulatable ingredients can be contained in the pressure-sensitive adhesive layer 3 of the plaster 1 of the embodiment, in addition to the above-mentioned readily dissolvable agar and additive.

The medicinal ingredients are not especially limited and can be suitably selected according to the purposes. In the plaster 1 of the embodiment, volatile ingredients and thermally decomposing ingredients can be used as medicinal ingredients and cosmetic ingredients. For formulating a common agar to prepare a pressure-sensitive adhesive base, a high temperature (about not less than 90°C) would have to be kept for fully dissolving the agar, so if thermally weak volatile ingredients or thermally decomposable medicinal ingredients and cosmetic ingredients were formulated concurrently, these ingredients would be greatly lost. When a pressure-sensitive adhesive base is prepared using a readily dissolvable agar, since the agar can be dissolved at a low temperature, the above-mentioned volatile ingredients and thermally decomposable ingredients can be used concurrently, easily providing a plaster having a good marketability. Further, since these ingredients can be directly formulated in a pressure-sensitive adhesive base containing an agar, also the simplification of the manufacturing process is possible.

The volatile ingredients are ingredients volatilizing by being given heat (for example, heat on manufacture). They include, for example, terpenes such as eucalyptus oils, myristica oils, thiamine oils, mentha oils, menthol, camphors, pinene, borneol and limonene, herb essential oils such as lavender, peppermint, Japanese mint, geranium, lime and chamomile, lower alcohols such as ethyl alcohol and isopropyl alcohol, flavoring agents such as fragrances and flavors, medicinals and cosmetics such as amyl nitrite and trimethadione, fatty acids of C8-C22 such as isostearic acid, octanoic acid and oleic acid, aliphatic alcohols of C8-C22, such as oleyl alcohol and lauryl alcohol, lower alkyl esters of fatty acids of C8-C22 such as ethyl oleate, isopropyl myristate, butyl stearate, and methyl laurate, dialkyl esters of dicarboxylic acids of C6-C8 such as diisopropyl adipate, monoglycerides of fatty acids of C8-C22 such as glycerine monolaurate, and liquid excipients such as tetrahydrofurfuryl alcohol polyethylene glycol ethers, polyethylene glycols, propylene glycols, 2-(2-ethoxy ethoxy) ethanol, diethylene glycol monomethyl ether and mixtures thereof.

These formulation amounts are different depending on the formulation ingredients, but for example, menthol is preferably 0.005% to 5.0% by mass to the total mass of a pressure-sensitive adhesive layer, more preferably 0.01 to 2.0% by mass. With the formulation proportion of menthol of not less than 0.005% by mass, the medicinal effect of menthol can be fully obtained; with that of not more than 5.0% by mass, the bleeding from the pressure-sensitive adhesive layer 3 can be prevented.

The thermally decomposable ingredients refer to substances unstable to heat (for example, heat on manufacture). They include, for example, non-steroid antiphlogistic anodynes having a carboxyl group such as diclofenac, felbinac, indomethacin and ketoprofen, proteins such as prostaglandins and albumin, toxins such as diphtheria toxin, enzymes such as catalase, peptides such as cyclosporin A, hirudine, somatostatin and thymopentin, hormones such as estrogen, peptide hormones such as human growth hormone, swine growth hormone, bovine growth hormone, human calcitonin, salmon calcitonin, carbocalcitonin and insulin, neurotransmitters such as antagonists of hormones and acetylcholine, glycoproteins such as antagonists of neurotransmitters and hyaluronic acid, lipoproteins such as α-lipoprotein, immunoglobulins such as IgG, immunomodulators such as interferon and interleukin, receptors of cells such as estrogen receptor proteins, glucosaminoglycans such as non-fractionated heparin, low-molecular heparin, chondroitin-4-sulfuric acid, chondroitin-6-sulfuric acid and derivatives thereof, prostaglandin, vegetable extracts such as ginseng, arnica and calendula, yeasts such as selenium yeast and beer yeast, animal proteins such as ovalbumin and gelatin, vegetable proteins such as gluten, hydrolyzed animal or vegetable proteins such as hydrolyzed collagen and hydrolyzed wheat gluten, natural polysaccharides such as hyaluronic acid, lecithins, collagens, placenta extracts, ascorbic acid and its esters, retinols and its esters, vitamins such as vitamin A oils, hydroxocobalamin acetate and ergocalciferol, isosorbide nitrate, nitroglycerol, testosterone enanthate, ergotamine tartrate, pergolide, nicotine, amyl nitrite, ethchlorvynol, paramethadione, and scopolamine.

The formulation amount is, for example, in the case of using ketoprofen, preferably 0.01 to 5.0% by mass, more preferably 0.1 to 1.0% by mass. With the formulation amount of ketoprofen of not less than 0.01 % by mass, the medicinal effect of ketoprofen can be fully obtained; with that of not more than 5.0% by mass, an excessive formulation amount relative to the desired medicinal effect can be prevented.

Ingredients having medicinal effects other than the above-mentioned volatile and thermally decomposable ingredients include, for example, nonsteroid anti-inflammatory agents such as flurbiprofen, spurofen, loxoprofen, piroxicam, methyl salicylate and glycol salicylate, steroid anti-inflammatory agents such as hydrocortisone, dexamethasone, fluocinolone acetonide, fludroxycortide, betamethasone valerate, acetic acid clobetasone and clobetasol propionate, thermosensitive substandes such as capsicum pepper extracts and nonyl acid vanillylamide, and antihistamines such as diphenhydramine hydrochloride and chlorphenylamine maleate.

The formulation amount is, for example, in the case of using betamethasone valerate, preferably 0.001 to 0.5% by mass, more preferably 0.005 to 0.2% by mass. With the formulation amount of betamethasone valerate of not less than 0.001% by mass, a sufficient medicinal effect can be obtained; with that of not more than 0.5% by mass, too strong a side effect can be prevented.

As water, purified water, sterilized water, natural water or the like can be used. These waters function as a dispersant and a solvent of a readily dissolvable agar, a water-soluble polymer and other ingredients, and water itself has an effect of improving the feeling in use and after use of the plaster 1. The formulation amount of water is preferably 30 to 95% by mass to the total mass of a pressure-sensitive adhesive layer, more preferably 45 to 65% by mass.

Polyhydric alcohols include glycerol, polyethylene glycols, 1,3-butylene glycol, propylene glycol, dipropylene glycol, sorbitol and xylitol, and particularly glycerol is preferable. The formulation amount of a polyhydric alcohol is preferably 10 to 60% by mass, more preferably 20 to 40% by mass. With the formulation proportion of not less than 10% by mass, the decrease in workability and the high cost can be prevented; by contrast, with that of not more than 60% by mass, the form preservability can easily be kept.

Polyvalent metal salts include, for example, aluminum hydroxide, aluminum hydroxide gel, hydrated aluminum silicate, kaolin, aluminum acetate, aluminum lactate, aluminum stearate, calcium chloride, magnesium chloride, aluminum chloride, dihydroxyaluminum aminoacetate, magnesium aluminometasilicate and magnesium aluminosilicate. Among them, particularly dihydroxyaluminum aminoacetate, synthetic aluminum silicate and magnesium aluminometasilicate are preferable.

The formulation amount of the polyvalent metal salt is preferably 0.01 to 1% by mass to the total mass of a pressure-sensitive adhesive layer, more preferably 0.02 to 0.5% by mass. With the formulation proportion of not less than 0.01% by mass, the gel strength can be made sufficient by fully progressing the reaction. By contrast, with that of not more than 1% by mass, a phenomenon that too high a reaction rate on manufacture brings about an inhomogeneous gelation, a decreased workability and an insufficient adhesiveness of a pressure-sensitive adhesive layer, can be prevented.

Surfactants include, for example, anionic surfactants such as sodium dioctylsulfosuccinate, alkyl sulfates, 2-ethylhexylalkylsulfuric acid ester sodium salts and sodium normal dodecylbenzenesulfonate; cationic surfactants such as hexadecyltrimethylammonium chloride, octadecyldimethylbenzyl ammonium chloride and polyoxyethylene dodecylmonomethylammonium chlorides; polyoxyethylene monostearates such as polyoxyethylene stearyl ethers, polyoxyethylene hydrogenated castor oils, polyoxyethylene tridecyl ethers, polyoxyethylene nonylphenyl ethers, polyoxyethylene octylphenyl ethers and monostearic acid polyethylene glycols; and nonionic surfactants such as sorbitan monostearate, sorbitan monopalminate, sorbitan sesquioleate, polyoxyethylene sorbitan monolaurates, polyoxyethylene sorbitan monooleates, glycerol monostearates, polyglyceryl fatty acid esters and polyoxyethylene octadecylamines.

Among them, monostearic acid polyethylene glycols, polyoxyethylene hydrogenated castor oils, monooleic acid and polyoxyetyhylene sorbitan are preferable.

The formulation amount of the surfactant is preferably 0.01 to 5% by mass to the total mass of a pressure-sensitive adhesive layer, more preferably 0.1 to 2% by mass. With the formulation proportion of not less than 0.01% by mass, the occurrence of bleeding can be prevented; by contrast, with that of not more than 5% by mass, the form preservability can easily be kept.

Further in the pressure-sensitive adhesive base of the embodiment, as required, skin-beautifying ingredients, humectants, antioxidants, crosslinking agents, preservatives, tackifiers, solvents, pigments, perfumes, ultraviolet absorbents, inorganic fillers and pH controlling agents, can be formulated.

The skin-beautifying ingredients include aloe extracts, rose fruit extracts, orange extracts, rasberry extracts, kiwi extracts, cucumber extracts, gardenia extracts, chamomile extracts, hawthorn extracts, juniper extracts, zizyphi fructus extracts, Duke extracts, tomato extracts, luffa extracts, succinyl kefiran, maleyl kefiran, malt root extracts, bara extracts, collagens, ceramides, squalane, hyaluronic acid, allantoin, horse chestnut extracts, water-soluble placental extracts, vitamin A, vitamin Bs, vitamin C, vitamin D, and vitamin E and its derivative.

The skin-beautifying ingredients other than the above include galenical ingredients such as moutan cortex, puerariae radix, peony root, angericae radix, cnidium rhizome, clove, swertiae herba, atractylodis lanceae, aurantii nobilis pericarpium, atractylodis rhizoma, aurantii percarpium, cinnamomi cortex, coptidis, phellodendri cortex and ginkgo, and mud ingredients such as bentonite, montmorillonite, saponite and hectorite.

The humectants include, for example, acylated kefiran aqueous solutions, malt extracts and glycols. These can be used singly or in combination of two or more.

The antioxidants include ascorbic acid, propyl gallate, butylhydroxyanisole, dibutylhydroxytoluene, nordihydroguaiaretic acid, tocopherol, tocopherol acetate, natural vitamin E, sodium nitrite and sodium hydrogen nitrite.

The crosslinking agents include hardly water-soluble aluminum compounds, thermosetting resins such as polyfunctional epoxy compounds, amino resins, phenol resins, epoxy resins, alkyd resins and unsaturated polyesters, isocyanate compounds, block isocyanate compounds, organic crosslinking agents, and inorganic crosslinking agents of a metal, a metal compound or the like. These can be used singly or in combination of two or more.

The preservatives include ethyl parahydroxybenzoate, propyl paraoxybenzoate, butyl paraoxybenzoate, sorbic acid and dehydroacetic acid.

The tackifiers include, for example, caseins, pullulans, dextrans, sodium alginate, soluble starches, carboxystarches, dextrins, carboxymethylcelluloses, sodium carboxymethylcelluloses, methyl celluloses, ethylcelluloses, hydroxyethylcelluloses, polyvinyl alcohols, polyethylene oxides, polyacrylamides, polyacrylic acids, polyvinylpyrrolidones, carboxyvinyl polymers, polyvinyl ethers, polymaleic acid copolymers, methoxyethylene maleic anhydride copolymers, isobutylene maleic anhydride copolymers, polyethyleneimines, partially saponified polyvinyl alcohols, hydroxypropylmethylcelluloses, xanthan gums and N-vinylacetamide.

The solvents include, for example, diethyl sebacate, N-methyl-2-pyrrolidone, methyl salicylate, glycol salicylate, triacetin, oleyl alcohol, benzyl alcohol, isopropyl myristate, diisopropyl adipate, crotamiton, mentha oil, camellia oil, castor oil and olive oil.

The pigments include, for example, Red No. 2 (Amaranthus), Red No. 3 (erythrosine), Red No. 102 (new coccine), Red No. 104-(1) (phloxine B), Red No. 105-(1) (rose bengal), Red No. 106 (acid red), Yellow No. 4 (Tartrazine), Yellow No. 5 (sunset yellow FCF), Green No. 3 (fast green FCF), Blue No. 1 (brilliant blue FCF) and Blue No. 2 (indigo carmine).

The perfumes include, for example, vegetable extracts such as mentha oil, cinnamon oil, clove oil, fennel oil, castor oil, turpentine, eucalyptus oil, orange oil, lavender oil, lemon oil, rose oil, lemon grass oil, rosemary and sage.

The ultraviolet absorbents include paraaminobenzoic acid, paraaminobenzoate, amyl paradimethylaminobenzoate, salicylate, menthyl anthranilate, umbelliferone, esculin, benzyl cinnamate, cinoxate, guaiazulene, urocanic acid, 2-(2-hydroxy-5-methylphenyl)benzotriazol, 4-methoxybenzophenone, 2-hydroxy-4-methoxybenzophenone, dioxybenzone, octabenzone, dihydroxydimethoxybenzophenone, sulisobenzone, benzoresorcinol, octyldimethyl paraaminobenzoate, ethylhexyl paramethoxycynamate and butylmethoxybenzoylmethane.

The inorganic fillers include, for example, calcium carbonate, magnesium carbonate, silicates (for example, aluminum silicate and magnesium silicate), barium sulfate, calcium sulfate, calcium plumbite, zinc oxide and titanium oxide.

The pH controlling agents include acetic acid, formic acid, lactic acid, tartaric acid, oxalic acid, benzoic acid, glycolic acid, malic acid, citric acid, hydrochloric acid, nitric acid, sulfuric acid, sodium hydroxide, calcium hydroxide, methylamine, ethylamine, propylamine, dimethylamine, diethylamine, dipropylamine, trimethylamine, triethylamine, tripropylamine, monomethanolamine, monoethanolamine, monopropanolamine, dimethanolamine, diethanolamine, dipropanolamine, trimethanolamine, triethanolamine, tripropanolamine, and buffer solutions such as a citric acid buffer, a glycine buffer, an acetic acid buffer and other buffer solutions.

The pH of the pressure-sensitive adhesive layer 3 of the plaster 1 of the embodiment can be optionally set in the range where the jelly strength of an agar does not decrease, but a pH of not more than 3 is unpreferable because the agar is liable to decompose at the pH. The pH is preferably about 4 to 10, more preferably 5 to 7.

The support 2 used for the plaster 1 of the embodiment is not limited as long as it can support the pressure-sensitive adhesive layer 3, and a stretchy or nonstretchy support can be used. The support specifically includes synthetic fibers or natural fibers such as polyurethanes, polyesters, polypropylenes, polyvinyl acetates, polyvinylidene chlorides, polyethylenes, polyethylene terephthalates, aluminum sheets, nylons, acryls, cottons, rayons and acetates, or fiber sheets as woven fabrics or nonwoven fabrics obtained by compositing these fibers, and further fiber sheets composed of a composite material thereof with a film having steam permeability or the like.

Among them, in view of safety, versatility and stretchability, a fiber sheet of a woven fabric or a nonwoven fabric composed of polyester, polyethylene or polyethylene terephthalate is more preferable. Such a fiber sheet, even if a thick fiber sheet, has flexibility and easily follows the skin, and has a low skin irritation. Further, use of such a fiber sheet allows to provide a plaster having a moderate self-supportability.

Further, the pressure-sensitive adhesive layer 3 of the plaster 1 of the embodiment includes the release sheet 4, on the surface thereof opposite to the support 2 and contacting with the skin. The release sheet 4 include, for example, films of polyester such as polyethylene terephthalate, polyvinyl chloride, polyvinylidene chloride or the like, and laminate films of quality paper and polyolefin or the like. The release sheet 4 is preferably one whose surface contacting with the pressure-sensitive adhesive layer 3 has been subjected to a silicone treatment. The silicone treatment allows the release sheet 4 to be easily peeled off the pressure-sensitive adhesive layer 3 in use.

The shape of the plaster 1 of the embodiment has no limitation, and may be not only of an ordinary rectangle or a rectangle with rounded four corners, but of a circle or an ellipse as well as a shape having holes or cut lines. Further, the shape may be one suitably designed according to a region of a body on which the plaster 1 is used. For example, it includes a shape obtained by cutting the plaster into a face shape so as to be pasted on the face and suitably cutting the shape for the eye, nose, mouth and jaw.

In manufacture of the plaster 1 of the embodiment, the gelation temperature of a solution containing a readily dissolvable agar is decreased due to addition of additives. Hence, even if a vessel or apparatus to be used is not specially kept warm, the time and the sol state necessary for applying the solution on the support 2 can be maintained, whereby the plaster is easily manufactured.

Specifically, ingredients such as a readily dissolvable agar, additives and medicinal ingredients are added to purified water at 65 to 75°C, and stirred until the readily dissolvable agar is dissolved. The prepared pressure-sensitive adhesive base is applied on a support 2 to form a coating, and a release sheet 4 is laminated on the coating. The coating is kept for several days to crosslink and form a pressure-sensitive adhesive layer 3. After the pressure-sensitive adhesive layer 3 is formed, the laminate is cut into a predetermined shape to obtain the plaster 1 of the embodiment.

Alternatively, after the prepared pressure-sensitive adhesive base is applied on the release sheet 4 to form the pressure-sensitive adhesive layer 3, the support 2 may be laminated on the pressure-sensitive adhesive layer 3.

The pressure-sensitive adhesive base, having been shown in the embodiment, having a specific composition containing a readily dissolvable agar and additives in specific ratios and amounts has a reduced gelation temperature and does not cause gelation until the pressure-sensitive adhesive base is applied on a support 2 or the like on manufacture. Consequently, the gelation, at undesired times during manufacture, of the pressure-sensitive adhesive base and the plaster 1 can be prevented without heating the manufacturing facility.

Further, the obtained plaster 1 has an excellent moisture preservability, form preservability and adhesive force, and exhibits reduced bleeding of the pressure-sensitive adhesive layer and sufficiently reduced residual of the pressure-sensitive adhesive layer.

Hereinbefore, the preferable embodiment of the plaster of the present invention and the manufacturing method of the plaster of the present invention have been described, but the scope of the present invention is not limited to the above-mentioned embodiment.

### Examples

### (Example 1)

0.5 part by mass of UP-37K (trade name, Ina Food Industry Co., Ltd., its jelly strength: 700 g/cm²) as a readily dissolvable agar, 0.5 part by mass of a synthetic aluminum silicate, 0.5 part by mass of monostearic acid polyethylene glycol as a surfactant and 3.0 parts by mass of a polyvinyl alcohol were added to warm water of 70 to 80°C, and stirred while kept at 70°C to dissolve the raw materials. Then, the total amount of the solution was transferred to a cooled mixer; then, a mixture composed of 0.5 part by mass of magnesium alminometasilicate, 0.3 part by mass of sodium edetate, 1.0 part by mass of L-menthol, 5.0 parts by mass of a partially neutralized polyacrylic acid and 30.0 parts by mass of glycerol was added thereto; further, purified water was added till the total amount became 100 parts by mass. The mixed liquid was cooled to and stirred at about 10°C to prepare a pressure-sensitive adhesive base. When the solution of the readily dissolvable agar during manufacture was transferred to the cooled mixer, the gelation state of the agar was observed, but no gelation was observed. The ingredients and the formulation proportions of the prepared pressure-sensitive adhesive base are shown in Table 1. Numerics in Table 1 are expressed by percent by mass.

**[Table 1]**

| | Example 1 | Example 2 | Example 3 | Example 4 | Example 5 |
|---|---|---|---|---|---|
| Readily dissolvable agar (700g/cm²) | 0.5 | 0.3 | 1.0 | 3.0 | 5.0 |
| Partially neutralized polyacrylic acid | 5.0 | 4.0 | 3.0 | 3.5 | 3.0 |
| Polyacrylic acid | | 2.0 | 3.0 | 2.0 | 1.0 |
| Polyvinyl alcohol | 3.0 | 2.5 | 4.0 | 5.0 | 5.0 |
| Glycerol | 30.0 | 30.0 | 25.0 | 20.0 | 20.0 |
| Polyethylene glycol | | | | 5.0 | |
| Surfactant | 0.5 | 0.5 | 0.5 | 0.5 | 0.5 |
| Synthetic aluminum silicate | 0.5 | 0.5 | 0.5 | 0.5 | 0.5 |
| Magnesium alminometasilicate | 0.5 | 0.5 | 0.5 | 0.5 | 0.5 |
| EDTA | 0.30 | 0.30 | 0.30 | 0.30 | 0.30 |
| L-menthol | 1.00 | 1.00 | 1.00 | 1.00 | 1.00 |
| Camphor | | | 0.3 | | |
| Ketoprofen | | 0.3 | | | |
| Indomethacin | | | | 0.5 | |
| Glycol salicylate | | | 1.0 | | |
| Peppermint oil | | | | | |
| Purified water | Suitable amount | Suitable amount | Suitable amount | Suitable amount | Suitable amount |

Then, the obtained pressure-sensitive adhesive base was applied on a polyester-made support such that the application amount was 1,000 g/cm²; a release sheet was laminated on the applied pressure-sensitive adhesive base to form a pressure-sensitive adhesive layer (thickness: about 140 µm). Thereafter, the laminate was cut into 14 cm x 10 cm to obtain a plaster.

The obtained plaster was measured for the adhesive force of the pressure-sensitive adhesive layer according to JIS Z0237, the rolling ball tack test. The results are shown in Table 2.

**[Table 2]**

| | Example 1 | Example 2 | Example 3 | Example 4 | Example 5 |
|---|---|---|---|---|---|
| Agar liquid dissolving temperature (°C) | 70 | 70 | 70 | 70 | 70 |
| Mixer set temperature (°C) | 10 | 10 | 10 | 10 | 10 |
| Confirmation of gelation | No gelation | No gelation | No gelation | No gelation | No gelation |
| Paste kneading temperature (°C) | 45.2 | 44.8 | 41.7 | 42.5 | 43.8 |
| Bleeding | No bleeding | No bleeding | No bleeding | No bleeding | No bleeding |
| Tonguing | No tonguing | No tonguing | No tonguing | No tonguing | No tonguing |
| Adhesive force (ball tack value) (mm) | 30 | 26 | 30 | 35 | 38 |

### (Examples 2 to 5)

Pressure-sensitive adhesive bases were prepared as in Example 1, except for using compositions shown in Table 1 in preparing the pressure-sensitive adhesive bases. When the solutions of the readily dissolvable agar during manufacture were transferred to the cooled mixer, the gelation state of the agar was observed, but no gelation was observed. Plasters were obtained as in Example 1 by using the obtained pressure-sensitive adhesive bases. Further, the obtained plasters were evaluated as in Example 1. The results are shown in Table 2.

### (Comparative Examples 1 to 3)

Plasters were obtained as in Example 1, except for preparing pressure-sensitive adhesive bases each by mixing at 70°C a mixed liquid of an ingredient composition containing no polyvinyl alcohol as an additive as shown in Table 3 in preparing the pressure-sensitive adhesive base. In preparing the pressure-sensitive adhesive bases, when the solutions of the readily dissolvable agar during manufacture were transferred to the cooled mixer, the gelation of the agar was observed.

**[Table 3]**

| | Comparative Example 1 | Comparative Example 2 | Comparative Example 3 |
|---|---|---|---|
| Readily dissolvable agar (700g/cm²) | 0.5 | 1.0 | 5.0 |
| Partially neutralized polyacrylic acid | 5.0 | 4.0 | 3.0 |
| Polyacrylic acid | | 2.0 | 1.0 |
| Polyvinyl alcohol | | | |
| Glycerol | 10.0 | 25.0 | 20.0 |
| Polyethylene glycol | 10.0 | | |
| Surfactant | 0.5 | 0.5 | 0.5 |
| Synthetic aluminum silicate | 2.0 | 1.0 | 0.5 |
| Magnesium alminometasilicate | 0.5 | 0.5 | 0.5 |
| EDTA | 0.30 | 0.30 | 0.30 |
| L-menthol | | 1.00 | 1.00 |
| Camphor | | | |
| Ketoprofen | | 0.3 | |
| Indomethacin | | | 1.0 |
| Glycol salicylate | | | |
| Peppermint oil | 1.0 | | |
| Purified water | Suitable amount | Suitable amount | Suitable amount |

The obtained plasters were evaluated as in Example 1. The results are shown in Table 4.

**[Table 4]**

| | Comparative Example 1 | Comparative Example 2 | Comparative Example 3 |
|---|---|---|---|
| Agar liquid dissolving temperature (°C) | 70 | 70 | 70 |
| Mixer set temperature (°C) | 10 | 10 | 10 |
| Confirmation of gelation | Gelation | Gelation | Gelation |
| Paste kneading temperature (°C) | 40.7 | 41.5 | 44.7 |
| Bleeding | Present | Present | Present |
| Tonguing | Present | Present | Present |
| Adhesive force (ball tack value) (mm) | Not measurable | Not measurable | Not measurable |

As shown in Table 2, the plasters of Examples 1 to 5 have an excellent form preservability and a sufficient adhesive force. By contrast, as shown in Table 4, the plasters of Comparative Examples 1 to 3 gelatinized during manufacture, resulting in bleeding and tonguing observed, thus not enabling to measure the adhesive force.

### Industrial Applicability

According to the present invention, a plaster having a pressure-sensitive adhesive layer which dissolves at a lower temperature and does not gelatinize till a lower gelation temperature in manufacturing can be provided by a simpler and more concise means.

## Claims

1. A plaster comprising a support and a pressure-sensitive adhesive layer disposed on at least one surface of the support, wherein the pressure-sensitive adhesive layer contains a readily dissolvable agar and at least one additive selected from the group consisting of polyacrylamides, polyvinyl alcohols, polyvinyl pyrrolidones, sodium alginate, ammonium alginate, carboxymethylcelluloses, sodium carboxymethylcelluloses, methylcelluloses, gum arabics, tragacanth gums, and xanthene rubbers.

2. The plaster according to Claim 1, wherein the additive contains at least one selected from the group consisting of polyacrylamides, polyvinyl alcohols and polyvinylpyrrolidones.

3. The plaster according to Claim 1 or 2, wherein the pressure-sensitive adhesive layer contains 0.1 % by mass to 20% by mass of the additive based on the total mass of the pressure-sensitive adhesive layer.

4. The plaster according to any one of Claims 1 to 3, wherein the pressure-sensitive adhesive layer contains 0.1 % by mass to 5.0% by mass of a readily dissolvable agar based on the total mass of the pressure-sensitive adhesive layer.

5. The plaster according to any one of Claims 1 to 4, wherein a formulation ratio of the readily dissolvable agar (mass: A) and the additive (mass: B) is A:B=1:0.5 to 1:10.

6. The plaster according to any one of Claims 1 to 5, wherein the readily dissolvable agar has a jelly strength of 450 g/cm² to 1,000 g/cm².
